# EUROPEAN PATENT APPLICATION

(11) **EP 1 930 019 A1**
(43) Date of publication of application: **11.06.2008**
(21) Application number: 06024384.7
(22) Date of filing: 24.11.2006
(51) Int. Cl.: A61K 36/53, A61K 127/00

(54) **Rosemary extracts, dietary and pharmaceutical compositions containing them and their uses**

(71) Applicant: DSM IP Assets B.V., 6411 TE Heerlen (NL)
(72) Inventor: The designation of the inventor has not yet been filed
(74) Representative: Steck, Melanie

(57) **Abstract**

The present invention refers to rosemary extracts for use as medicament for the treatment of a disorder connected to impaired neurotransmission, as well as to dietary and pharmaceutical compositions and their uses.

## Description

The present invention refers to rosemary extracts for use as medicaments, especially for the treatment of disorders connected to impaired neurotransmission, as well as to dietary and pharmaceutical compositions containing such rosemary extracts and their uses.

It is well known that impaired neurotransmission, e.g. low neurotransmitter levels, is connected to mental diseases such as depression.

Compounds that increase neurotransmitter levels in the brain and thus enhance their transmission, exhibit therefore antidepressant properties as well as beneficial effects on a variety of other mental disorders ("Neurotransmitters, drugs and brain function" R.A. Webster (ed), John Wiley & Sons, New York, 2001, p. 187-211, 289-452, 477-498). The main neurotransmitters are serotonin, dopamine, norepinephrine, noradrenaline, acetylcholine, glutamate, gamma-amino-butyric acid, as well as neuropeptides. Increase in neurotransmission is achieved by increasing the concentration of the neurotransmitter in the synaptic cleft thus making it available for increased or prolonged neurotransmission through inhibition of re-uptake into the pre-synaptic nerve end, or by preventing neurotransmitter catabolism by inhibition of degrading enzymes such as monoaminooxidase A and B.

The term "impaired neurotransmission" is used in the present application in accordance with its meaning well-known to the person skilled in the art and relates to a deregulation of neurotransmission and which may occur at the level of neurotransmitter biosynthesis, processing, storage, release re-uptake and receptor binding. Impaired neurotransmission may manifest itself in animals including humans as a disturbance of behaviour, emotions, mood and thinking processes, for example, in one of various types of depression.

Tricyclic antidepressant compounds (TCAs) such as imipramine, amitriptyline, and clomipramine e.g. inhibit the re-uptake of serotonin and noradrenaline. They are widely regarded as among the most effective antidepressants available, but they have a number of disadvantages because they interact with a number of brain receptors, e.g. with cholinergic receptors. Most importantly, TCAs are not safe when taken in overdose, frequently showing acute cardiotoxicity.

Another class of antidepressant drugs are the so-called SSRIs (selective serotonin re-uptake inhibitors) including fluoxetine, paroxetine, sertraline, citalopram, fluvoxamine that block the serotonin transporter (SERT), a high affinity sodium chloride-dependent neurotransmitter transporter that terminates serotonergic neurotransmission by uptake of serotonin. They have been proven as effective in the treatment of depression and anxiety, and are usually better tolerated than TCAs. These medications are typically started at low dosages and may be increased until they reach a therapeutic level. A common side effect is nausea. Other possible side effects include decreased appetite, dry mouth, sweating, infection, constipation, yawn, tremor, sleepiness and sexual dysfunction.

In addition, compounds that prevent the catabolism of neurotransmitters more broadly by inhibiting the monoaminooxidases (MAOs) A and B exhibit antidepressant effects. MAOs catalyse the oxidation of amino group containing neurotransmitters such as serotonin, noradrenaline, and dopamine.

Furthermore, modulators of neurotransmission exert pleiotropic effects on mental and cognitive functions.

There is a need for compounds for the treatment or prevention of mental diseases and/or disorders which do not show the negative side effects of known antidepressants. Many patients are interested in alternative therapies which could minimize the side effects associated with high-dose of drugs and yield additive clinical benefits. Severe depression is a long lasting and recurring disease, which is usually poorly diagnosed. Furthermore many patients suffer from mild or middle severe depression. Thus, there is an increasing interest in the development of compounds as well as pharmaceutical and/or dietary compositions that may be used to treat mental diseases/disorders or to prevent the development of mental diseases/disorders such as depression in people at risk, and to stabilize mood.

According to the present invention this demand is met with rosemary extracts, especially with those rosemary extracts that are manufactured by a process comprising the following steps:
- grinding dried leaves of Rosmarinus officinalis,
- extracting the grinded rosemary leaves with acetone to obtain an acetone extract,
- optionally (preferably) filtrating the acetone extract,
- concentrating the acetone extract by (partly, preferably nearly completely) removing the acetone,
- optionally (preferably further) spray-drying the thus concentrated acetone extract.

The thus manufactured rosemary extract contains > 35 weight-% of carnosic acid, preferably > 40 weight-% of carnosic acid, preferably > 50 weight-% of carnosic acid, based on the total weight of the rosemary extract Thus manufactured rosemary extracts are especially suitable for the purposes of the present invention.

Rosemary extracts also especially suitable for the purposes of the present invention contain rosmanol (preferably (*4*α*R, 9S, 10*α*S)*-rosmanol) (preferably in the range of from 0.05 to 3 weight-%, more preferably in the range of from 0.1 to 1.0 weight-%, most preferably in the range of from 0.2 to 0.7 weight-%), carnosol (preferably) (preferably in the range of from 0.5 to 7 weight-%, more preferably in the range of from 1 to 5,0 weight-%, most preferably in the range of from 2.5 to 4.0 weight-%), carnosic acid (preferably (*4*α*R,10*α*S*)-carnosic acid) (preferably in the range of from 20 to 65 weight-%, more preferably in the range of from 25 to 60 weight-%, most preferably in the range of from 30 to 55 weight-%) and carnosic acid 12-methyl ether (preferably (*4*α*R,10*α*S*)-carnosic acid 12-methyl ether) (preferably in the range of from 1 to 20 weight-%, more preferably in the range of from 5 to 15 weight-%, most preferably in the range of from 7 to 12 weight-%), based on the total weight of the rosemary extract and measured by HPLC-UV (High Pressure Liquid Chromatography-Ultraviolet) at 210 nm with the pure substances as reference.

"Carnosol" means the racemic mixture as well as pure (*4*α*R,9S,10*α*S*)-carnosol or pure (*4*α*S,9R,10*α*R*)-carnosol or any mixture or diastereoisomer of them. (*4*α*R,9S,10*α*S*)-Camosol is preferred.

"Carnosic acid" means the racemic mixture as well as pure (*4aR,10aS*)-carnosic acid or pure (*4*α*S,10*α*R*)-carnosic acid or any mixture or diastereoisomer of them. Preferred is (*4*α*R,10*α*S*)-carnosic acid.

"Carnosic acid 12-methyl ether" means the racemic mixture as well as pure (*4*α*R*,*10*α*S*)-carnosic acid 12-methyl ether or pure (*4*α*S*,*10*α*R*)-carnosic acid 12-methyl ether or any mixture or diastereoisomer of them. Preferred is *(4*α*R, 10*α*S*)-carnosic acid 12-methyl ether.

"Rosmanol" means the racemic mixture as well as pure (*4*α*R*,*9S*,*10*α*S*)-rosmanol or pure (*4*α*S,9R*,*10*α*R*)-rosmanol or any mixture or diastereoisomer of them. (*4*α*R,9S,10*α*S*)-Rosmanol is preferred.

Thus, in one aspect the invention relates to rosemary extracts with the preferences as cited above for use as medicament for the treatment of a disorder connected to impaired neurotransmission.

In another aspect, the invention relates to the use of rosemary extracts as defined above for the manufacture of a composition for the treatment of a disorder connected to impaired neurotransmission, particularly for the manufacture of an antidepressant, a mood improver, a stress reliever and/or a condition improver.

In still another aspect, the invention relates to a dietary composition containing a rosemary extract as defined above as well as to a pharmaceutical composition containing a rosemary extract as defined above and a conventional pharmaceutical carrier.

Further, the invention relates to a method for the treatment of a disorder connected to impaired neurotransmission in animals including humans, said method comprising administering an effective dose of a rosemary extract as defined above to animals including humans which are in need thereof.

Animals in the context of the present invention include humans and encompass mammals, fish and birds, Preferred "animals" are humans, pet animals and farm animals.

Examples for pet animals are dogs, cats, birds, toy fish, guinea pigs, (jack) rabbits, hares and ferrets. Examples for farm animals are fish, pigs, horses, ruminants (cattle, sheep and goat) and poultry.

According to the present invention not only the rosemary extracts themselves, with the preferences as given above, but also dietary and pharmaceutical compositions containing them can be used as medicament, especially for the treatment of a disorder connected to impaired neurotransmission.

The dietary compositions according to the present invention may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellyfying agents, gel forming agents, antioxidants and antimicrobials.

The term "dietary compositions" comprises any type of (fortified) food/feed and beverages including also clinical nutrition, and also dietary supplements.

Beside a pharmaceutically acceptable carrier and a rosemary extract as defined above, the pharmaceutical compositions according to the present invention may further contain conventional pharmaceutical additives and adjuvants, excipients or diluents, including, but not limited to, water, gelatin of any origin, vegetable gums, ligninsulfonate, talc, sugars, starch, gum arabic, vegetable oils, polyalkylene glycols, flavoring agents, preservatives, stabilizers, emulsifying agents, buffers, lubricants, colorants, wetting agents, fillers, and the like. The carrier material can be organic or inorganic inert carrier material suitable for oral/parenteral/injectable administration.

The dietary and pharmaceutical compositions according to the present invention may be in any galenic form that is suitable for administrating to the animal body including the human body, especially in any form that is conventional for oral administration, e.g. in solid form such as (additives/supplements for) food or feed, food or feed premix, fortified food or feed, tablets, pills, granules, dragées, capsules, and effervescent formulations such as powders and tablets, or in liquid form such as solutions, emulsions or suspensions as e.g. beverages, pastes and oily suspensions. The pastes may be filled into hard or soft shell capsules, whereby the capsules feature e.g. a matrix of (fish, swine, poultry, cow) gelatin, plant proteins or ligninsulfonate. Examples for other application forms are forms for transdermal, parenteral or injectable administration. The dietary and pharmaceutical compositions may be in the form of controlled (delayed) release formulations.

Examples for fortified food are cereal bars, bakery items such as cakes and cookies.

Beverages encompass non-alcoholic and alcoholic drinks as well as liquid preparations to be added to drinking water and liquid food. Non-alcoholic drinks are e.g. soft drinks, sport drinks, fruit juices, lemonades, teas and milk based drinks. Liquid food are e.g. soups and dairy products.

The rosemary extracts as defined above can be used for the manufacture of medicaments for the treatment of a disorder connected to impaired neurotransmission.

In the context of this invention "treatment" also encompasses co-treatment as well as prevention. "Prevention" can be the prevention of the first occurrence (primary prevention) or the prevention of a reoccurence (secondary prevention).

In the context of this invention the term "disorder" also encompasses diseases.

Medicaments for the treatment of disorders connected to impaired neurotransmission encompass antidepressants, mood improvers, stress relievers and condition improvers. They all improve, enhance and support the physiological neurotransmission, especially in the central nervous system, and therefore alleviate mental malfunction.

Antidepressants are medicaments for treating mental, behavioural and emotional/affective, neurotic, neurodegenerative, eating and stress related disorders such as e.g. unipolar depression, bipolar depression, acute depression, chronic depression, subchronic depression, dysthymia, postpartum depression, premenstrual dysphoria/syndrom (PMS), climacteric depressive symptoms, aggression, attention deficit disorders (ADS), social anxiety disorders, seasonal affective disorders, anxiety (disorders), fibromyalgia syndrome, chronic fatigue, sleep disorders (insomnia), post-traumatic stress disorders, panic disorders, obsessive compulsive disorders, restless leg syndrome, nervousness, migraine/primary headaches and pain in general, emesis, bulimia, anorexia nervosa, binge eating disorder, gastrointestinal disorders, bum out syndrome, irritability and tiredness.

Antidepressants can also be used for (the manufacture of compositions for) primary and secondary prevention and/or the treatment of neurocognitive impairment. Furthermore they are also effective in the treatment of depressive symptoms or other symptoms related to disturbed neurotransmission occurring as comorbidity in chronic diseases such as cardiovascular diseases, strokes, cancer, Alzheimer disease, Parkinson disease, and others.

The rosemary extracts as defined above as well as dietary/pharmaceutical compositions containing them are thus suitable for the treatment of animals including humans.

Especially pet animals and farm animals can be in conditions in need of enhanced or improved neurotransmission. Such conditions e,g, occur after capture or transport or with housing, when the animals develop analogous disorders and are distressed or aggressive, or display stereotypic behaviour, or anxiety and obsessive-compulsive behaviour.

Thus, the rosemary extracts as defined above can be used in general as antidepressants for animals including humans, preferably for humans, pet animals and farm animals.

In a further embodiment of the present invention the rosemary extracts as defined above find use as mood improver in general as well as for the manufacture of compositions for such use (dietary/pharmaceutical compositions). "Mood improver " means that the mood of a person treated with it is enhanced, that the self esteem is increased and/or that the negative thoughts are reduced. It also means the emotions are balanced and/or that the general well being is improved.

Moreover, rosemary extracts as defined above as well as compositions comprising an effective dose of them are useful for the treatment and prevention of stress, for the alleviation of stress related symptoms, for the increase of the resistance or tolerance to stress and/or to favor and facilitate the relaxation in normal healthy individuals i.e. such compositions have an effect as "stress reliever".

A further embodiment of the present invention relates to the use of rosemary extracts as defined above and to the use of compositions containing them (dietary/pharmaceutical compositions) as "condition improver", i.e. as means to reduce irritability and tiredness, to favour undisturbed sleep and to increase energy in more general terms in diseased or normal healthy individuals. Moreover for cognition improvement in general, for the regulation of hunger and satiety as well as for the regulation of motor activity.

The present invention not only refers to rosemary extracts as defined above and their compositions (dietary/pharmaceutical compositions containing them) for use as medicaments, especially for the treatment of disorders connected to impaired neurotransmission, but also for the methods for the treatment of such disorders themselves, as already mentioned above.

In an especially preferred embodiment of such method pet animals or farm animals whose disorders are associated with housing, capture or transport are treated and which may appear in form of anxiety or obsessive-compulsive behaviour.

For humans a suitable daily dosage of rosemary extracts for the purposes of the present invention may be within the range of from 0.001 mg per kg body weight to 100 mg per kg body weight per day. More preferred is a daily dosage in the range of from 0.01 to 10 mg per kg body weight, and especially preferred is a daily dosage in the range of from 0.05 to 5.0 mg per kg body weight.

In solid dosage unit preparations for humans, the rosemary extracts are suitably present in an amount in the range of from 0,1 mg to 1000 mg, preferably in the range of from 1 mg to 500 mg per dosage unit.

In dietary compositions, especially in food and beverages for humans, the rosemary extracts are suitably present in an amount in the range of from 0.0001 (1 mg/kg) to 5 weight-% (50 g/kg), preferably in the range of from 0.001 % (10 mg/kg) to 1 weight-% (10 g/kg), more preferably in the range of from 0.01 (100 mg/kg) to 0.5 weight-% (5 g/kg), based upon the total weight of the food or beverage.

In food and drinks in a preferred embodiment of the invention the amount of rosemary extracts are in the range of from 10 to 30 mg per serving, i.e. e.g. ca. 120 mg per kg food or drink.

For animals excluding humans a suitable daily dosage of rosemary extracts, for the purposes of the present invention may be within the range of from 0.001 mg per kg body weight to 1000 mg per kg body weight per day. More preferred is a daily dosage in the range of from 0.1 mg to 500 mg per kg body weight, and especially preferred is a daily dosage in the range of from 1 mg to 100 mg per kg body weight.

For pet animals the same daily dosages as for humans are suitable.

The invention is illustrated further by the following examples.

### Examples

### Example 1: Preparation of the rosemary extract

Dried leaves of Rosmarinus officinalis were grinded and then extracted with acetone. The thus obtained acetone extract was filtered and concentrated by removing the acetone. The concentrated acetone extract was then spray-dried.

The thus produced rosemary extract ("Oxy'less" (No. 993 369) from Naturex, Avignon, France) contained 0.5 weight-% of rosmanol, 3.3 weight-% of carnosol, 36.7 weight-% of carnosic acid and 9.0 weight-% of carnosic acid 12-methyl ether, based on the total weight of the rosemary extract and measured by HPLC-UV (High Pressure Liquid Chromatography-Ultraviolet) at 210 nm with the pure substances as reference.

### Example 2: Serotonin uptake inhibition by rosemary extract.

HEK-293 cells stably expressing the human serotonin re-uptake transporter (hSERT) were obtained from R. Blakely, Vanderbilt University, USA. The cells were routinely grown in Dulbeco's Modified Eagles Medium (Bioconcept) containing 10% fetal calf serum, penicillin, streptomycin, L-glutamine and the antibiotic G418 and passaged by trypsinisation. On the day of assay, cells from 80% confluent flasks were harvested by gentle washing with warm phosphate buffered saline (PBS). Cells were then washed once by centrifugation and re-suspended in Krebs Ringers bicarbonate buffer (Sigma) supplemented with 35 µM pargyline, 2.2 mM CaCl₂, 1 mM ascorbic acid and 5 mM N-2-hydroxyethylpiperaaine-N'-2-ethanesulfonic acid (buffer called "Hepes") at a concentration of 10,000 cells in 160 ul of buffer, and aliquoted into round bottomed polypropylene 96 well microtitre plates (Coming) at 10,000 cells per well, Serotonin uptake into the cells was determined by addition of radio-labeled (3H) serotonin (GE Healthcare) to a concentration of 20 nM, and incubation for 40 minutes at 37°C with gentle shaking. At the end of this time unincorporated label was removed by filtration though Unifilter 96 GF/B plates (Perkin Elmer) using a Tomtec Mach III M cell harvester. The incorporated serotonin retained on the plates was quantified by liquid scintillation counting using Microscint-40 / Topcount (Perkin Elmer).

The effect of the rosemary extract obtained according to example I on the serotonin uptake was determined by its inclusion in the assay at a range of concentrations between 0.03 and 100 µM for 10 minutes prior to and during the addition of (3H) serotonin, Serotonin uptake via the transporter was inhibited by the rosemary extract in a concentration dependent manner. The calculated IC₅₀ values for inhibition of serotonin uptake by the rosemary extract are shown in Table 1.

**Table 1: Inhibition of serotonin uptake into transfected HEK-293 cells by rosemary extract**

| **Substance** | **IC₅₀** for Tritiated Serotonin Uptake [µM unless otherwise stated] |
|---|---|
| Rosemary Extract according to example 1 | 6.9 ug/ml +/-1.4 s,e.m. n = 2 |

The experiment was carried out twice (n = 2) so that the average value +/- the standard error in the mean (s.e.m.) is shown.

### Example 3: Monoaminooxidase inhibition by rosemary extract

The organic amines p-tyramine or benzylamine were used as substrates for the Monoamine oxidase A (MAO-A) and B (MAO-B) enzymes respectively. The H₂O₂ produced by this reaction was quantified by reaction with vanillic acid, catalysed by horse radish peroxidase (HRP).

The reactions were carried out in polystyrene microtitre plates. The MAO enzymes (final concentration 2 U/ml) were mixed with either p-tyramine (Sigma, final concentration 0.5 mM) or benzylamine (Sigma, final concentration 0.5 mM) as appropriate and the chromogenic solution (containing vanillic acid (Fluka), 4-aminoatipyrine (Fluka) and horse radish peroxidase (Sigma), final concentrations 0.25 mM, 0.125 mM and 1 U/ml respectively) in 0.2 M potassium phosphate buffer pH 7.6. The reactions were followed in a microtitre plate absorbance reader e.g. Spectramax M5 (Molecular Devices Corporation). Absorbance readings at 495 nm were taken every 15 seconds for 40 minutes and the initial reaction velocities calculated by linear regression using SOFTmaxPro (Molecular Devices Corporation).

The effect of the rosemary extract on the monoamine oxidase enzymes was determined by their inclusion in the assay at a range of concentrations between 0.03 and 100 µM for 10 minutes prior to and during the incubation with substrate. To determine the effect of the extracts on the HRP catalyzed portion of the reaction, the MAO enzyme was replaced by H₂O₂ (Molecular Probes, final concentration 0.2 mM). The reactions containing MAO-A and MAO-B were both inhibited by the rosemary extract in a concentration dependent manner, whilst the control reaction, was unaffected. The measured IC50 values for inhibition of monoamine oxidase activity by the rosemary extract are shown in Table 2.

**Table 2:Inhibition of MAO-A and MAO-B by the rosemary extract according to example 1.**

| **Substance** | **IC50 for Inhibition of MAO-A [µM unless otherwise stated]** | **IC50 for Inhibition of MAO-B [µM unless otherwise stated]** |
|---|---|---|
| Rosemary Extract according to example 1 | 0.93 ug/ml +/- 0.13 s.e.m. n = 2 | 3.0 ug/ml +/- 0.4 s.e.m. n = 2 |

The main value of the two IC₅₀ determinations +/- the standard error in the mean (s.e.m.) is shown.

### Example 4: Effects of Rosemary extract in Porsolt's forced swim test in the mouse after subchronic, oral gavage

The "Behavioural Despair Test" or "Porsolt's Forced Swim Test" is a validated animal model for depression (see T. Nagatsu, NeuroToxicology 2004, 25, 11-20, especially p. 16, right column in the middle, in combination with Porsolt et al., Arch. Int. Pharmacodyn. 1977, 229, 327-336). It responds to enhancement of the transmission of several neurotransmitters including serotonin, dopamine and noradrenaline.

The test, which detects antidepressant activity, was carried out similar to the protocol described by Porsolt et al. in Arch. Int. Pharmacodyn. 1977, 229, 327-336. Mice which are forced to swim in a situation from which they cannot escape rapidly become immobile. Antidepressants decrease the duration of immobility.

The rosemary extract was evaluated at 3 doses: 272, 409, 545 mg/kg body weight administered orally 24, 5, and 1 hour before the test, and compared with a vehicle group. The amounts of each constituent compound, as included in the extract, are displayed in the table below.

| Compound/extract | % present (w/w) | Dose administered (mg/kg) | | | | |
|---|---|---|---|---|---|---|
| total extract | | 27* | 81* | 272 | 409 | 545 |
| rosmanol | 0.5 | 0.14 | 0.4 | 1.4 | 2 | 2.7 |
| carnosol | 3.3 | 0.9 | 2.7 | 9 | 13.5 | 18 |
| camosic acid | 36.7 | 9.9 | 29.7 | 100 | 150 | 200 |
| carnosic acid-12-methyl-ether | 9.0 | 2.4 | 7.3 | 24.5 | 36.8 | 49 |

| | | | | | | |
|---|---|---|---|---|---|---|
| * doses used in microdialysis studies (see Example 5, below) | | | | | | |

The thus administered rosemary extract was dissolved in tocopherol-stripped corn oil (so called "vehicle"). The positive control group were administered imipramine (dissolved in water) at 32 mg/kg body weight and the vehicle group were administered to copherol-stripped corn oil, orally at 24, 5, and 1 hour before the swim test.
Mice were individually placed in a cylinder (Height = 24.5 cm, Diameter = 19.5 cm) containing 13.5 cm water (22°C) from which they could not escape. The mice were placed in the water for 6 minutes, monitored by the VideoMot2 system and the duration of immobility during the last 4 minutes was measured,
10 mice were studied per each of the five groups. Data were analyzed by comparing the treated groups and the positive control group with the vehicle group using Analysis of Variance (ANOVA) and the Bonferroni post-hoc test,

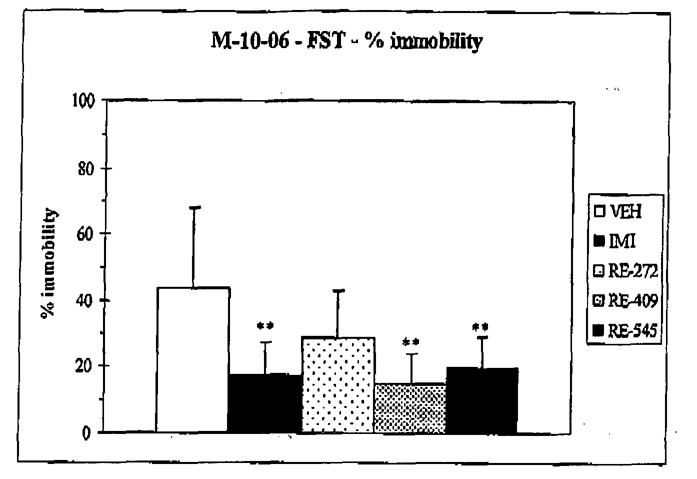

Data shown as % immobility (i.e. duration of immobility calculated as a % of test time). Mean ± S,D. (n = 9-10), **p < 0.01

The results show that rosemary extract demonstrated significant antidepressant activity, which is similar to that of imipramine.

### Example 5: Microdialysis analysis for serotonin and noradrenaline (NA)

Male Sprague-Dawley rats (250-320g) were housed in groups of 4-5 under conditions of controlled temperature (21 ± 2°C) and humidity (55 ± 10%) with free access to food and water (lights on 07.00-19.00). Rats were anaesthetised using chloral hydrate (400mg/kg IP) and a single microdialysis probe (BASi type MD2200, 2mm membrane, 30,000 dalton cutoff) was implanted in the dorsal hippocampus using a stereotaxic frame at the following coordinates (rostro-caudal -4.5mm; medio-lateral -2.5mm ; dorso-ventral -4.5mm from bregma and dura surface according to Paxinos & Watson 6) and fixed in position with dental cement. Body temperature was maintained at 36°C using a heating pad and monitored via a digital rectal thermometer. The microdialysis probe was perfused with artificial cerebrospinal fluid (aCSF) at 1 ml/min and extracellular monoamine levels determined by collection of perfusate samples every 15min and assayed using high-performance liquid chromatography (HPLC) with electrochemical detection. The HPLC mobile phase (0.5mM EDTA, O.1M monochloroacetic acid pH 3.1, 0.15g/L sodium octyl sulphate, 5% acetonitrile, 0.7% tetrahydrofuran) was pumped through the system at 70 µl/min. Monoamines were separated using a reverse-phase 1 x 100mm ODS 3 µm microbore column with 5 µl injection loop and detected using a Epsilon electrochemical detector (BASi) with a glassy carbon electrode set at +650mV versus Ag/AgCl reference electrode. Dialysate peaks were identified by comparing peak elution times with reference standards and quantified according to measurement of peak area using linear regression analysis. The detection limits for 5HT and 5HIAA were defined as the sample amount producing a peak area twice that of the background noise per unit time and were approximately 0.1 fmol/sample in both cases.

Rosemary extract was injected intraperitoneally in saline + 0.2% hydroxypropylmethylcellulose, at lml/kg volume, at doses of 27 & 81 mg/kg (see Example 4, above, for the amount of each of the main constituent compounds included in these doses of the extract).

Preliminary studies demonstrated that following implantation of the dialysis probe, the 5HT level was initially high due to release from platelets activated by blood clotting caused by the surgery but within 150min of completion of the surgery the basal 5HT level was almost constant. Injections were therefore routinely administered at 150, 210 and 270 minutes following the surgery and perfusate samples collected until 60 minutes following the final injection. For routine assay, two rats were prepared of which one was used to test the rosemary extract and the other a control (vehicle) sample. Because of the variability in the basal release of 5HT between assays and variability in the efficiency of the microdialysis probes to detect 5HT in the extracellular fluid, these data were normalised according to the 2 values obtained immediately prior to the first injection (namely samples at 135 and 150 min time-points). Data from replicate studies for each compound are therefore expressed as % Basal level, as is normal practice for microdialysis studies. To determine the effect of each dose of the extract on the total amount of 5HT released, the area-under-the-curve (AUC) was estimated using the trapezoid method over the sampling period following each dose administration and values for the test compound / extract compared to the appropriate control by 2-way ANOVA (test factors being Treatment and Dose) followed by post-hoc comparisons at individual doses using the Banferroni t-test. All statistical analyses were carried out using Graphpad Prism.

Aliquots of the samples harvested for 5HT analysis were frozen for subsequent noradrenaline analysis.

Samples were thawed on ice and then assayed immediately for NA content using high-performance liquid chromatography (HPLC) with electrochemical detection.

The HPLC mobile phase (0.5mM EDTA, 0.1M NaH2PO4 pH 3.1, 1mM sodium octyl sulphate, 6% acetonitrile) was pumped through the system at 70 µl/min. Monoamines were separated using a reverse-phase I x 100mm ODS 3 µm microbore column with 5 µl injection loop and detected using a Epsilon electrochemical detector (BASi) with a glassy carbon electrode set at +65OmV versus Ag/AgCl reference electrode. Dialysate peaks were identified by comparing peak elution times with reference standards and quantified according to measurement of peak area using linear regression analysis. The detection limit for NA was defined as the sample amount producing a peak area twice that of the background noise per unit time and was approximately 0.3 fmol/sample.

Data were normalised according to the 2 basal values obtained immediately prior to the first injection (namely samples at 135 and 150 min time-points) and are expressed as % Basal level.

Statistical comparison of the level of NA in the perfusate following administration of the test compound / extract compared to the appropriate control (either saline or hydroxypropylmethylcellulose vehicle) was made by 2-way ANOVA (test factors being Treatment and Time) followed by post-hoc comparisons at individual time-points using the Bonferroni t-test.

### Results

Following the lower dose there was no change in the NA level but following the higher dose there was a small (approximately 10%) but sustained increase in NA level compared to the vehicle control samples (n = 6). Comparison of NA levels following administration of rosemary extract or vehicle by 2-way ANOVA over the full period of investigation revealed a significant effect of treatment (F(1,128) = 17.63 ; p<0.001) but no significant interaction effect treatment x time (F(13, 128) = 1.13 ; p>0.05). Timed comparison of NA levels indicated a significantly higher NA level immediately following the higher dose but not at any subsequent time-point. The basal levels of NA in the assays of rosemary extract (20.7 ± 4.1 fmol/sample) were not significantly different from the levels in the vehicle control assays. Estimation of the cumulative NA release measured as AUC in the 90 min following administration of each dose of treatment as analysed by 2-way ANOVA indicated a significant overall effect of treatment (F(1,20) = 6.84 ; p<0.05) but no significant interaction effect treatment x time (F(1,20) = 1.24 ; p>0.05). Individual comparisons after each dose indicated significantly higher NA following rosemary extract at the higher dose only (p<0.05) compared to the corresponding vehicle samples.

To determine the effect of each dose of compound / extract on the total amount of NA. released, the area-under-the-curve (AUC) was estimated using the trapezoid method over the sampling period following each dose administration and values for the test compound / extract compared to the appropriate control by 2-way ANOVA (test factors being Treatment and Dose) followed by post-hoc comparisons at individual doses using the Bonferroni t-test. All statistical analyses were carried out using Graphpad Prism.

### Example 6: Effect of rosemary extract on cumulative 5HT release measured as AUC

in the 90 min following administration of each dose.

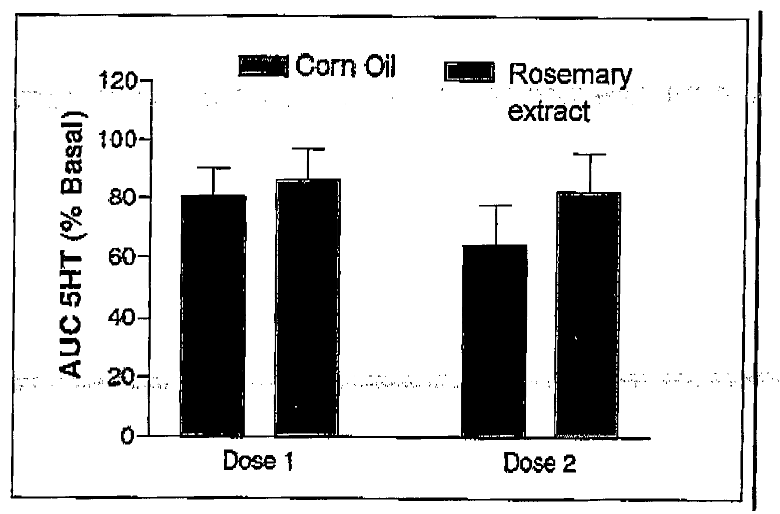

At each doses the left-handed column shows the AUC 5HT of corn oil, and the right-handed column the AUC 5HT of the rosemary extract.

Data expressed as % basal values. Mean ± s,e,m. (rosemary extract: n = 6, vehicle: n = 4) Although two-way ANOVA showed no significant difference, there was an indication that 5HT levels increased after injection of 81 mg/kg body weight

### Example 7: Effect of rosemary extract on extracellular NA level in rat dorsal hippocampus measured by in vivo microdialysis.

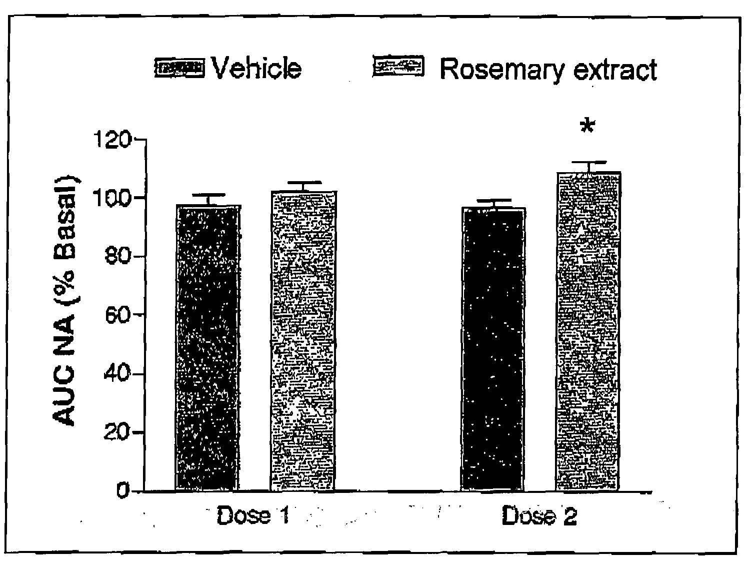

At each doses the left-handed column shows the AUC NA of the vehicle, and the right-handed column the AUC NA of the rosemary extract.

Effects of Rosemary extract on total NA release measured as AUC in the 90 min following administration of each dose.
Data expressed as % basal values. Mean s.e.m. (Rosemary extract: n = 6, vehicle: n = 6) Two-way ANOVA: significant overall effect of treatment, F(1,20) = 6.84; p < 0.05.

Thus, using *in vivo* microdialysis, the rosemary extract according to example I demonstrated a tendency to increase brain extracellular serotonin levels, while inducing significant increases in extracellular noradrenaline.

### Example 8: Preparation of a soft gelatin capsule

A soft gelatin capsule (500 mg) may be prepared comprising the following ingredients:

| **Ingredient** | **Amount per Capsule** |
|---|---|
| Rosemary extract | 500 mg |
| Lecithin | 50 mg |
| Soy bean oil | 250 mg |

Two capsules per day for 3 months may be administered to a human adult for the treatment of mild chronic dysthymia.

### Example 9: Preparation of a soft gelatin capsule

A soft gelatin capsule (600 mg) may be prepared comprising the following ingredients:

| **Ingredient** | **Amount per Capsule** |
|---|---|
| Rosemary extract | 200 mg |
| Evening prim rose oil | 300 mg |
| Vitamin B₆ | 100 mg |

One capsule per day preferably at the second half of the menstrual cycle may be taken for 14 days for the treatment of premenstrual syndrome and premenstrual dysphoric disorder.

### Example 10: Preparation of a tablet

A 400 mg-tablet may be prepared comprising the following ingredients:

| **Ingredient** | **Amount per tablet** |
|---|---|
| Rosemary extract | 100 mg |
| Passion flower standardized extract | 150mg |
| Green Tea Extract, e.g, TEAVIGO® from DSM Nutritional Products, Kaiseraugst, Switzerland | 150 mg |

For general well being, energizing and stress alleviation, one tablet may be taken twice daily for 3 months.

### Example 11: Preparation of an instant flavoured soft drink

| **Ingredient** | **Amount [g]** |
|---|---|
| Rosemary extract | 0.9 |
| Sucrose, fine powder | 922.7 |
| Ascorbic acid, fine powder | 2,0 |
| Citric acid anhydrous powder | 55.0 |
| Lemon flavour | 8.0 |
| Trisodium citrate anhydrous powder | 6.0 |
| Tricalciumphosphate | 5.0 |
| β-Carotene 1% CWS from DNP AG, Kaiseraugst, Switzerland | 0.4 |
| **Total amount** | **1000** |

All ingredients are blended and sieved through a 500µm sieve. The resulting powder is put in an appropriate container and mixed on a turbular blender for at least 20 minutes. For preparing the drink, 125 g of the obtained mixed powder are taken and filled up with water to one liter of beverage.
The ready-to-drink soft drink contains ca. 30 mg rosemary extract per serving (250 ml).
As a strenghtener and for general well being 2 servings per day (240ml) may be drunk.

### Example 12: Preparation of a fortified non baked cereal bar

| **Ingredient** | **Amount [g]** |
|---|---|
| Rosemary extract | 0.95 |
| Sugar | 114.55 |
| Water | 54.0 |
| Salt | 1.5 |
| Glucose syrup | 130.0 |
| Invert sugar syrup | 95.0 |
| Sorbitol Syrup | 35.0 |
| Palmkernel fat | 60.0 |
| Baking fat | 40.0 |
| Lecithin | 1.5 |
| Hardenend palm-oil | 2.5 |
| Dried and cut apple | 63.0 |
| Cornflakes | 100.0 |
| Rice crispies | 120.0 |
| Wheat crispies | 90.0 |
| Roasted hazelnut | 40.0 |
| Skim milk powder | 45.0 |
| Apple flavour 74863-33 | 2.0 |
| Citric acid | 5.0 |
| **Total amount** | 1000 |

Rosemary extract is premixed with skim milk powder and placed in a planetary bowl mixer. Cornflakes and rice crispies are added and the total is mixed gently. Then the dried and cut apples are added. In a first cooking pot sugar, water and salt are mixed in the amounts given above (solution 1). In a second cooking pot glucose, invert and sorbitol syrup are mixed in the amounts given above (solution 2). A mixture of baking fat, palmkernel fat, lecithin and emulsifier is the fat phase. Solution 1 is heated to 110°C. Solution 2 is heated to 113°C and then cooled in a cold water bath, Afterwards solution 1 and 2 are combined. The fat phase is melted at 75°C in a water bath. The fat phase is added to the combined mixture of solution 1 and 2. Apple flavour and citric acid are added to the liquid sugar-fat mix. The liquid mass is added to the dry ingredients and mixed well in the planetary bowl mixer. The mass is put on a marble plate and rolled to the desired thickness. The mass is cooled down to room temperature and cut into pieces. The non baked cereal bar contains ca. 25 mg rosemary extract per serving (30 g). For general well-being and energizing 1-2 cereal bars may be eaten per day.

### Example 13: Dry doe feed comprising rosemary extract for relieving stress and revitalizing the dog

Commercial basal diet for dogs (e.g. Mera Dog "Brocken", MERA-Tiemahrung GmbH, Marienstraße 80-84, D-47625 Kevelaer-Wetten, Germany) is sprayed with a solution of rosemary extract in an amount sufficient to administer to a subject a daily dose of 50 mg per kg body weight based on the weight of the rosemary extract. The food composition is dried to contain dry matter of about 90 % by weight. For an average dog of 10 kg body weight to consume approx. 200g dry feed per day, the dog food contains approx. 2500 mg rosemary extract/kg food, For heavier dogs, the feed mix is prepared accordingly.

### Example 14: Wet cat food comprising rosemary extract

Commercial basal diet for cats (e.g. Happy Cat "Adult", Tierfeinnahrung, Südliche Hauptstraße 38, D-86517 Wehringen, Germany) is mixed with a solution of rosemary extract in an amount sufficient to administer to a subject a daily dose of 100 mg per kg body weight based on the weight of the dried rosemary extract. For an average cat of 5 kg of body weight to consume approx. 400 g of wet food, the cat food contains 1250 mg/kg of rosemary extract. The food composition is dried to contain dry matter of about 90% by weight.

### Example 15: Dog treats containing rosemary extract

Commercial dog treats (e.g. Mera Dog "Biscuit" for dogs as supplied by Mera Tiernahrung GmbH, Marienstrasse 80-84, 47625 Kevelaer-Wetten, Germany) are sprayed with a solution of rosemary extract in an amount sufficient to administer to the treats 5 - 50 mg per g treats based on the weight of the dried rosemary extract. The food composition is dried to contain dry matter of about 90% by weight.

### Example 16: Cat treats containing rosemary extract

Commercial cat treats (e.g. Whiskas Dentabits for cats as supplied by Whiskas, Master-foods GmbH, Eitzer Str. 215, 27283 Verden/Aller, Germany) are sprayed with a solution of rosemary extract in an amount sufficient to administer to the treats 5 - 50 mg per g treats based on the weight of the dried rosemary extract. The food composition is dried to contain dry matter of about 90% by weight

## Claims

1. The acetone extract of rosemary leaves for use as medicament for the treatment of a disorder connected to impaired neurotransmission.

2. * The acetone extract according to claim 1 manufactured by a process comprising the following steps:
grinding dried leaves of Rosmarinus officinalis;
extracting the grinded rosemary leaves with acetone to obtain an acetone extract;
optionally (preferably) filtrating the acetone extract;
concentrating the acetone extract by (partly, preferably nearly completely) removing the acetone;
optionally (preferably further) spray-drying the thus concentrated acetone extract.

3. The acetone extract according to claim 1 wherein the acetone extract contains rosmanol (preferably in the range of from 0.05 to 3 weight-%), carnosol (preferably in the range of from 0.5 to 7 weight-%), carnosic acid (preferably in the range of from 20 to 65 weight-%) and camosic acid 12-methyl ether (preferably in the range of from 1 to 20 weight-%; all amounts based on the total weight of the acetone extract) and is preferably manufactured by a process according to claim 2.

4. The rosemary extract according to any of claims 1 to 3 for use as antidepressant, mood improver, stress reliever and/or condition improver.

5. Use of a rosemary extract according to any one of claims 1 to 4 for the manufacture of a composition for the treatment of a disorder connected to impaired neurotransmission.

6. The use according to claim 5, wherein the composition is an antidepressant, a mood improver, a stress reliever and/or a condition improver.

7. A dietary composition containing a rosemary extract as defined in any of claims 1 to 4.

8. A pharmaceutical composition containing a rosemary extract as defined in any of claims 1 to 4 and a conventional pharmaceutical carrier.

9. A method for the treatment of a disorder connected to impaired neurotransmission in animals including humans, said method comprising administering an effective dose of a rosemary extract as defined in any of claims 1 to 4 to animals including humans which are in need thereof.

10. The method according to claim 9, wherein the animal is a human, a pet animal or a farm animal.
